# FASCICULE DE BREVET EUROPEEN

(11) **EP 0 607 337 B1**
(45) Date de publication et mention de la délivrance du brevet: **09.04.1997**
(21) Numéro de dépôt: 92922336.0
(22) Date de dépôt: 07.10.1992
(51) Int. Cl.: C07H 21/02

(54) **PROCEDE DE SYNTHESE D'ACIDE RIBONUCLEIQUE (ARN) UTILISANT UN NOUVEAU REACTIF DE DEPROTECTION**
VERFAHREN ZUR SYNTHESE VON RNS MIT EINEM NEUEN SCHUTZGRUPPENABSPALTUNGSREAGENS
PROCESS FOR RNA SYNTHESIS USING A NEW DEPROTECTION REAGENT

(30) Priorité: 08.10.1991 FR 9112357
(43) Date de publication de la demande: 27.07.1994
(73) Titulaire: COMMISSARIAT A L'ENERGIE ATOMIQUE, 75015 Paris (FR); CIS BIO INTERNATIONAL, 91400 Saclay (FR)
(72) Inventeur: DUPLAA, Anne-Marie, F-38130 Echirolles (FR); GASPARUTTO, Didier, F-38400 Saint-Martin d'Hères (FR); LIVACHE, Thierry, F-38000 Grenoble (FR); MOLKO, Didier, F-38210 Tullins-Fures (FR); TEOULE, Robert, F-38000 Grenoble (FR)
(74) Mandataire: Des Termes, Monique
(86) Numéro de dépôt international: FR9200929
(87) Numéro de publication internationale: WO9307164

(56) Documents cités:
- WO-A-85/01051
- WO-A-90/15814
- DATABASE WPI Section Ch, Week 9119, 26 Juin 1991 Derwent Publications Ltd., London, GB; Class B, Page 21,

## Description

La présente invention a pour objet un procédé de synthèse d'acide ribonucléique (ARN) utilisant un réactif de déprotection qui permet d'obtenir rapidement une déprotection totale des groupes hydroxyles en 2' de l'ARN.

Depuis quelques années, les procédés de synthèse de fragments d'acide ribonucléique se sont développés énormément en raison de l'intérêt croissant porté à l'ARN qui joue un rôle important dans de nombreux processus cellulaires.

Ces procédés de synthèse qui sont proches des procédés de synthèse de l'acide désoxyribonucléique (ADN), ont été décrits par exemple par Ogilvie et al dans Proc. Natl. Acad. Sci., vol. 85, p. 5764-5768, Août 1988 et par Gasparutto et al dans Nucléosides & Nucléotides 9(8), p. 1087-1098, 1990.

Pour cette synthèse, on part de ribonucléosides dont l'hydroxyle en 2' est protégé par un groupement approprié et qui comportent par ailleurs des groupements convenant à la synthèse nucléotidique, et on condense successivement ces dérivés de nucléosides pour former l'acide ribonucléique.

Ainsi, Ogilvie et al dans le document précité, ont utilisé des dérivés de ribonucléosides dans lesquels les groupements hydroxyles en 2' sont protégés par le radical t-butyl-diméthylsilyle. Après couplage de tous les nucléosides, on obtient donc un acide ribonucléique dont les hydroxyles en 2' sont protégés. Par conséquent, il convient de réaliser en fin d'opération un traitement de déprotection pour éliminer ces groupements protecteurs des hydroxyles en 2'.

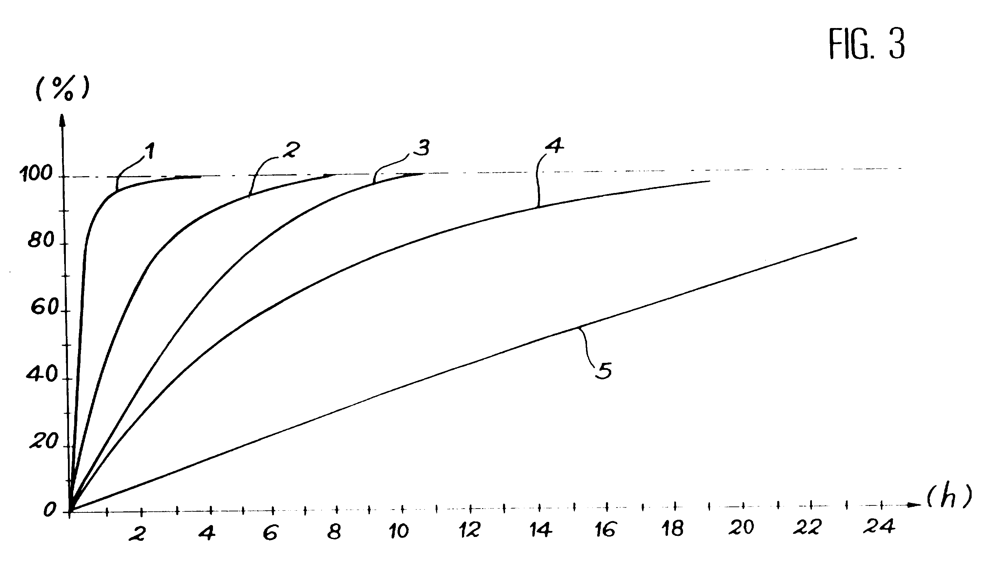

Jusqu'à présent, on a réalisé cette déprotection en utilisant comme réactif de déprotection du fluorure de tétrabutylammonium dans le tétrahydrofurane comme il est décrit dans Glen Research Report, vol. 4, n°1, mars 1991, RNA Synthesis - Problems in Deprotection. La déprotection de la fonction hydroxyle en 2' d'un nucléoside ou d'un nucléotide libre portant en 2' une fonction tertio-butyl dimetylsilyle (TBDMS) s'effectue dans le fluorure de tetrabutylammorium en quelques minutes. Elle est beaucoup plus difficile dans le cas d'un oligonucleotide portant t-butyldimethylsilyle en 2' : elle nécessite des heures (voir fig. 3, courbe 5). On a constaté que dans le cas de fragments d'ARN de grande longueur, par exemple de plus de 25 bases, la déprotection des groupes hydroxyles n'est pas complète avec ce réactif car il y a effectivement au moins 1 à 2% de groupes hydroxyles non déprotégés par unité de base, c'est-à-dire que chaque molécule d'ARN possède au moins un hydroxyle non déprotégé, ce qui peut nuire à l'activité biologique de l'ARN comme l'indique d'ailleurs Ogilvie.

La présente invention a précisément pour objet un procédé de synthèse d'acide ribonucléique (ARN) qui permet d'obtenir totalement et rapidement la déprotection totale des groupes hydroxyles en 2' de l'acide ribonucléique synthétisé.

Ce procédé consiste
a) à condenser successivement sur un premier nucléoside de formule : 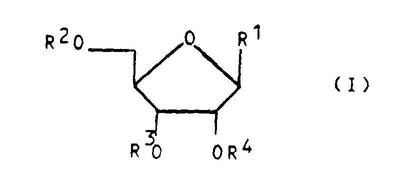 dans laquelle R¹ est un radical dérivé d'une base pyrimidique ou purique choisi parmi les racicaux de formule : 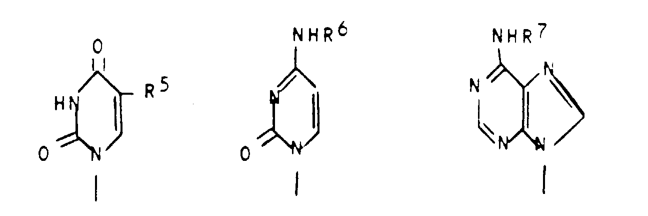 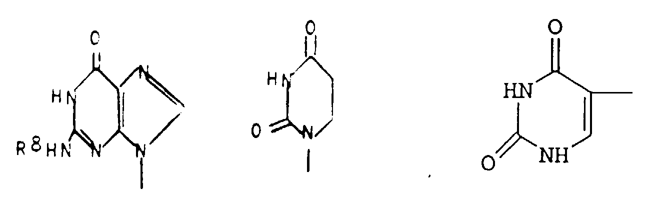 dans lesquelles
   R⁵ est H ou CH₃, et R⁶, R⁷ et R⁸ sont des atomes d'hydrogène ou un groupe acyle protecteur d'un groupement NH₂ exocyclique,
   R² est un radical labile en milieu acide adapté à la synthèse nucléotidique,
   R³ est un support solide, et
   R⁴ est un radical trialkylsilyle,
   un ou plusieurs nucléosides identiques ou différents répondant à la formule (I) dans laquelle R¹, R² et R⁴ ont la signification donnée ci-dessus et R³ est un radical phosphoré adapté à la synthèse nucléotidique, pour former un acide ribonucléique synthétique dont tous les groupements hydroxyles en 2' sont protégés par R⁴, et
b) à déprotéger tous les hydroxyles en 2' par traitement de l'acide ribonucléique ainsi synthétisé avec un réactif de déprotection répondant à la formule : 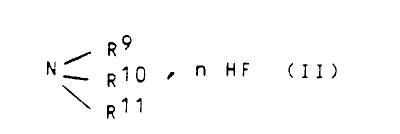 dans laquelle R⁹ représente un radical alkyle, linéaire ou ramifié en C₁ à C₁₀, R¹⁰ et R¹¹ qui peuvent être identiques ou différents, représentent H ou un radical alkyle, linéaire ou ramifié, en C₁ à C₁₀, identique ou différent de R⁹, et n est un nombre allant de 1 à 3, entier ou non.

Dans ce procédé, on réalise les cycles successifs de condensation pour coupler les différents nucléosides entre eux en utilisant des méthodes classiques, c'est-à-dire les protocoles et les réactifs chimiques prescrits par les fournisseurs de synthétiseurs d'ARN.

Dans ce but, le nucléoside de formule (I) utilisé pour chaque couplage comprend des radicaux R² et R³ appropriés à la synthèse nucléotidique et un groupement R¹ qui dépend de la base utilisée. Lorsque cette base comporte un groupement NH₂ exocyclique, celui-ci est protégé par les groupes protecteurs appropriés utilisés généralement pour la synthèse nucléotidique.

Des groupes protecteurs appropriés sont les groupes acyle décrits par exemple dans les documents suivants : US-A- 4 980 460, Koster et al, Tetrahedron, vol. 37, p. 363-369, 1981 ; Wu et al, Nucleic Acids Research, vol. 17, 9, 1989, p. 3501-3517.

De préférence, dans l'invention, lorsque la base est l'adénine ou la guanine, on utilise comme groupe protecteur le groupe phénoxyacétyle, c'est-à-dire que R⁷ et R⁸ dans les formules précitées sont de préférence le groupe phénoxyacétyle.

Lorsque la base est la cytosine, on utilise de préférence comme groupe protecteur, le groupement acétyle, c'est-à-dire que R⁶ dans la formule précitée représente un groupement acétyle. On peut aussi utiliser dans ce cas le groupement isobutyryle ou propionyle.

Pour R², les radicaux labiles en milieu acide susceptibles de convenir sont par exemple :
- les radicaux trityle répondant à la formule : 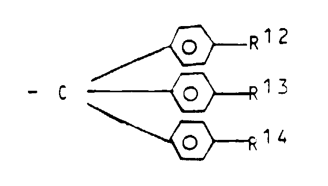 dans laquelle R¹², R¹³ et R¹⁴ qui peuvent être identiques ou différents, représentent un atome d'hydrogène, un radical alkyle ou un radical alkoxy, par exemple le radical monométhoxytrityle, diméthoxytrityle,
- les radicaux pixyle, et
- les radicaux phényl-9 xanthényle.

Pour R³, les radicaux phosphorés susceptibles d'être utilisés peuvent être par exemple les radicaux de formule : 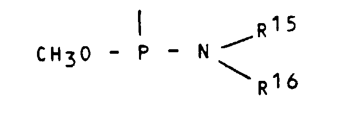 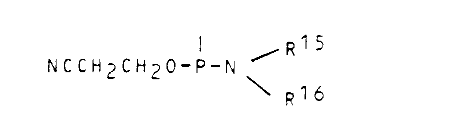 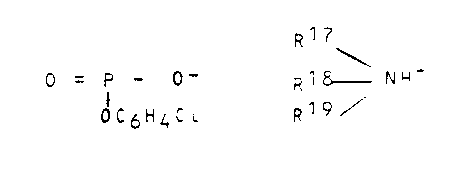 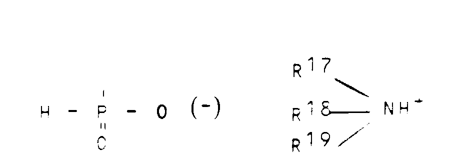 dans lesquelles R¹⁵, R¹⁶, R¹⁷, R¹⁸ et R¹⁹ sont des radicaux alkyle, linéaires ou ramifiés.

De préférence, dans le procédé de l'invention, on utilise pour R³ des groupements phosphoramidites de formule : 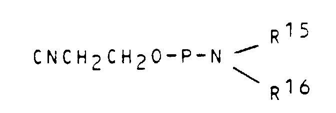 dans laquelle R¹⁵ et R¹⁶ qui sont identiques ou différents, sont des radicaux alkyle en C₁ à C₄.

A titre d'exemple, R¹⁵ peut être le radical méthyle et R¹⁶ le radical éthyle, ou R¹⁵ et R¹⁶ peuvent être tous deux des radicaux isopropyle.

L'emploi des radicaux méthyle et éthyle pour R¹⁵ et R¹⁶ est très intéressant car on obtient avec ceux-ci un couplage rapide des nucléotides (formation de la liaison internucléotidique en 3 minutes) avec un excellent rendement (98%) et moins de réactions parasites. De plus, l'utilisation de ces radicaux avec le groupe protecteur t-butyl diméthylsilyle en 2' conduit à des dérivés de nucléosides stables ayant une réactivité excellente pour la synthèse d'ARN, alors que ces radicaux éthyle et méthyle donnent des dérivés très instables pour la synthèse d'ADN.

Selon le procédé de l'invention, après avoir réalisé les cycles successifs de condensation des nucléosides, on soumet l'acide ribonucléique synthétique dont tous les hydroxyle en 2' sont protégés à un traitement de déprotection en utilisant comme réactif de déprotection, le réactif répondant à la formule : 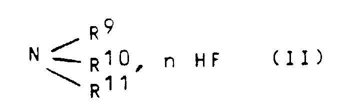 dans laquelle R⁹ représente un radical alkyle, linéaire ou ramifié, en C₁ à C₁₀, R¹⁰ et R¹¹ qui peuvent être identique ou différents, représentent H ou un radical alkyle, linéaire ou ramifié, en C₁ à C₁₀, identiques ou différent de R⁹, et n est un nombre allant de 1 à 3, entier ou non.

A titre d'exemples de tels réactifs, on peut citer N(C₂H₅)₃, 3HF ; N(CH₃)₃, 2HF ; (CH₃)₂NH, HF ; isopropylamine, HF ; isopropylamine, 1,8 HF ; diisopropylamine, 2 HF et diisopropylamine, 3 HF.

De préférence, dans l'invention, on utilise le réactif de déprotection répondant à la formule :

N(C₂H₅)₃, 3HF

Ce réactif connu a été utilisé jusqu'à présent comme réactif de fluoration des hydrates de carbone.

Selon l'invention, l'emploi de ce réactif pour la déprotection des hydroxyles en 2' de l'acide ribonucléique est très intéressant car il permet d'obtenir une déprotection totale d'un acide ribonucléique de très grande longueur en une dizaine d'heures.

Par ailleurs, il peut être utilisé pur et être ensuite éliminé facilement par simple évaporation.

Généralement, on réalise ce traitement de déprotection à la température ambiante, pendant des durées allant de 2 à 20h.

On peut aussi utiliser ce réactif dilué dans un solvant organique, par exemple dans de l'acétonitrile.

D'autres caractéristiques et avantage de l'invention apparaîtront mieux à la lecture des exemples suivants donnés bien entendu à titre illustratif et non limitatif, en référence au dessin annexé sur lequel
- la figure 1 représente l'ARN de transfert préparé dans l'exemple 1,
- la figure 2 représente les phosphoramidites de ribonucléosides utilisés pour la synthèse de l'ARN de transfert de l'exemple 1,
- la figure 3 est un diagramme représentant en fonction du temps (en h) le pourcentage de déprotection d'un oligonucléotide de 42 bases, par différents réactifs de déprotection.

Les figures 4 à 9 sont des chromatogrammes d'oligonucléotides obtenus lors d'une chromatographie liquide à haute performance.

### Exemple 1 : Synthèse de l'acide ribonucléique de transfert t-ARN de l'alanine de E. coli K₁₂.

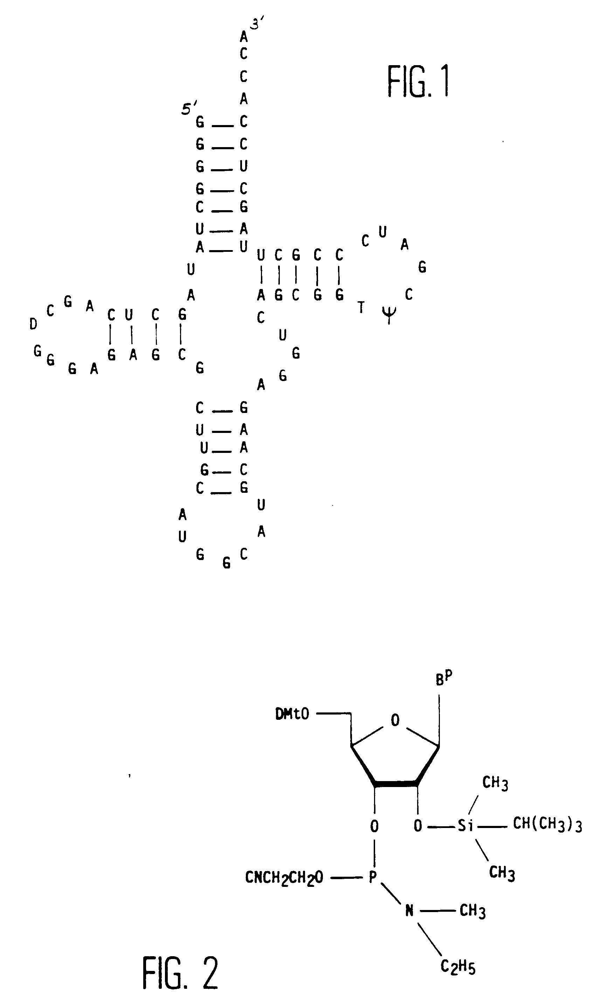

Cet ARN de transfert est illustré sur la figure 1.

Pour réaliser cette synthèse, on a utilisé les phosphoramidites de ribonucléosides protégés, représentés sur la figure 2. Sur cette figure DMt représente le groupement diméthoxytrityle et B^{P} représente la base purique ou pyrimidique, éventuellement protégée. Dans le cas où la base est l'adénine ou la guanine, son groupement NH₂ exocyclique est protégé par le groupe phénoxyacétyle, lorsqu'il s'agit de la cytosine, le groupement NH₂ exocyclique est protégé par le groupe acétyle.

La base B^{P} peut être aussi l'uracile, la thymine, la 5,6 dihydrouracile, et la pseudouracile.

On réalise les étapes successives de condensation des nucléosides avec un synthétiseur, en utilisant l'anhydride phénoxyacétique comme réactif de blocage (capping).

La synthèse est réalisée en phase solide à une échelle de 0,2µmol, en utilisant un support de gel de silice à dimensions de pores controlées.

Chaque cycle de condensation comporte les étapes suivantes :
- détritylation : acide trichloracétique à 2% dans CH₂Cl₂ pendant 1min30,
- lavage : CH₃CN pendant 2min,
- condensation : dérivé de nucléoside + agent d'activation dans CH₃CN anhydre pendant 3min,
- masquage des fonctions hydroxyles n'ayant pas réagi pour stopper l'élongation des chaînes incomplètes : mélange d'anhydride phénoxyacétique et de méthyl-1 imidazole dans CH₃CN anhydre pendant 1min,
- oxydation : iode à 0,45% dans tétrahydrofurane/eau/lutidine (89,5/10/0,5) pendant 45 secondes,
- lavage : CH₃CN pendant 1min30s.

Dans chaque cyle, on utilise 45 équivalents de nucléoside.

En fin d'opération, on libère l'oligonucléotide du support et on hydrolyse le groupe cyanoéthyle par traitement au moyen d'un mélange d'éthanol et d'une solution aqueuse d'ammoniaque concentrée (ammoniaque 28% : éthanol, 3/1 en volume) pendant 2h à la température ambiante.

On chauffe ensuite l'oligonucléotide à 55°C pendant 1h pour éliminer les groupes protecteurs des groupes NH₂ exocycliques des bases, puis on évapore la solution sous vide et on maintient le résidu à la température ambiante dans 300µl du réactif de déprotection N(C₂H₅)₃, 3HF pur (TEA, 3HF) pendant 16h.

On évapore ensuite la phase liquide à sec et on dessale le mélange de produits, puis on purifie l'acide ribonucléique synthétique obtenu par électrophorèse sur gel de polyacrylamide à 15% (1,5mm d'épaisseur).

On vérifie ensuite, après marquage des extrémités OH en 3' ou en 5' par du phosphore 32, que l'acide ribonucléique obtenu correspond bien à l'ARN de la figure 1, en réalisant un séquençage par des procédés de dégradation enzymatique.

Dans tous les cas, la structure de l'acide ribonucléique de transfert est en accord avec la séquence donnée sur la figure 1.

On vérifie encore la structure de l'acide ribonucléique synthétique ainsi obtenu, en réalisant sur une petite quantité de cet ARN une hybridation de son extrémité 3' avec un oligodésoxynucléotide complémentaire d'une longueur de 16 bases et copie avec la transcriptase inverse. Après amplification PCR, les deux chaines d'ADN peuvent être séquencées et elles correspondent parfaitement à la séquence attendue.

### Exemple 2.

Dans cet exemple, on évalue précisément le taux de déprotection de l'invention sur un oligonucléotide dT₂₁rUdT₂₀, servant de modèle, qui est un oligonucléotide comportant une chaîne d'ADN dans laquelle est inclus un seul ribonucléotide rU protégé par un groupe tertiobutyl diméthylsilyle.

Pour synthétiser cet oligonucléotide dT₂₁rUdT₂₀, on suit le même mode opératoire que dans l'exemple 1, sauf que l'on utilise comme nucléosides de départ le composé de la figure 2 dans lequel B^{P} représente la thymine et dans lequel 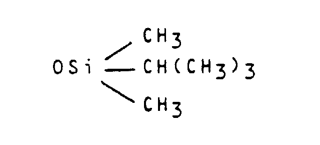 est remplacé par H, et le composé de la figure 2 dans lequel B^{P} représente l'uracile.

Après synthèse, on réalise la déprotection pendant des durées différentes, et après chaque traitement de déprotection, on détermine le taux de déprotection en séparant le produit obtenu sur une colonne C₁₈ à phase inversée, ce qui permet de séparer les fragments complètement déprotégés de leurs homologues portant encore le groupement tertiobutyl diméthylsilyle.

Les résultats obtenus sont représentés par la courbe (1) de la figure 3.

Sur cette figure, on voit que l'on obtient un taux de déprotection de 100% en 4h.

### Exemple 3.

On suit le même mode opératoire que dans l'exemple 2 pour préparer l'oligonucléotide dT₂₁-rU-dT₂₀ mais on réalise la déprotection en utilisant un mélange du réactif de l'invention (TEA, 3HF) et d'acétonitrile avec un rapport en volume de 1/1. Les résultats obtenus sont représentés par la courbe 2 de la figure 3.

Sur cette figure, on voit que le fait de diluer le réactif de déprotection de l'invention par l'acétonitrile, permet d'obtenir une déprotection complète au bout de 8h.

### Exemple comparatif 1.

Dans cet exemple, on suit le même mode opératoire que dans l'exemple 2 pour préparer l'oligonucléotide dT₂₁-rU-dT₂₀ mais on réalise la déprotection en utilisant le réactif de déprotection de l'art antérieur, soit le fluorure de tétrabutylammonium trihydraté, (TBAF, 3H₂O) à 1mol/l dans du diméthylsulfoxyde.

Les résultats obtenus sont représentés par la courbe 3 de la figure 3.

Ainsi, avec le réactif de l'art antérieur, il faut 11h pour obtenir une déprotection totale.

### Exemple comparatif 2.

Dans cet exemple, on suit le même mode opératoire que dans l'exemple 2 pour préparer l'oligonucléotide dT₂₁-rU-dT₂₀, mais on utilise pour la déprotection, une solution à 1mol/l de TBAF, 3H₂O dans du diméthylformamide.

Les résultats obtenus sont représentés par la courbe 4 de la figure 3.

On remarque que dans ce cas, il faut attendre 24h pour obtenir la déprotection totale.

### Exemple comparatif 3.

Dans cet exemple, on suit le même mode opératoire que dans l'exemple 2 pour préparer l'oligonucléotide dT₂₁-rU-dT₂₀ et on réalise la déprotection en utilisant une solution à 1mol/l de TBAF, 3H₂O dans du tétrahydrofurane (THF).

Les résultats obtenus sont représentés par la courbe 5 de la figure 3.

Au vu de cette figure, on remarque qu'il est impossible d'obtenir une déprotection totale avec le fluorure de tétrabutylammonium dans THF, qui est le réactif actuellement utilisé.

### Exemple 4.

Dans cet exemple, on suit le même mode opératoire que dans l'exemple 1 pour préparer un oligonucléotide de 18 bases de long, ayant la séquence suivante :

5'UTUACUAUCUAUCUCCAA3'

Après élimination des groupes protecteurs des groupes NH₂ exocycliques des bases en milieu ammoniacal, on traite environ 40µg soit 2 unités d'absorption à 260nm (20D) de l'oligonucléotide silylé dans un microtube Eppendorf par 60µl du réactif de déprotection N(C₂H₅)₃, 3HF (TEA, 3HF) pendant 20h à la température ambiante. On prélève des parties aliquotes du mélange réactionnel pour suivre l'évolution de la réaction, avant la réaction avec TEA, 3HF, après 4h de réaction et après 20h de réaction. Après prélèvement, on neutralise chaque partie aliquote par 4 volumes d'acétate de triéthyl ammonium TEAA (pH 7,0, 25mM), puis on l'analyse par chromatographie liquide à haute performance en échange d'ions en utilisant une colonne mono Q (LKB) et en éluant la colonne par un gradient de 0 à 1% KCl en 40min dans du tampon KH₂PO₄, (pH 6,0). Dans le cas du prélèvement effectué après 20h de réaction, une partie de celui-ci est analysé comme les prélèvements précédents après neutralisation, et l'autre partie de celui-ci est dessalé, après neutralisation par TEAA, sur une colonne de Sephadex G25 (NAP 10 de Pharmacia), puis analysé par chromatographie liquide à haute performance (HPLC) de la même façon. Le dessalage permet d'éliminer les pics parasites engendrés par le mélange TEA, 3HF.

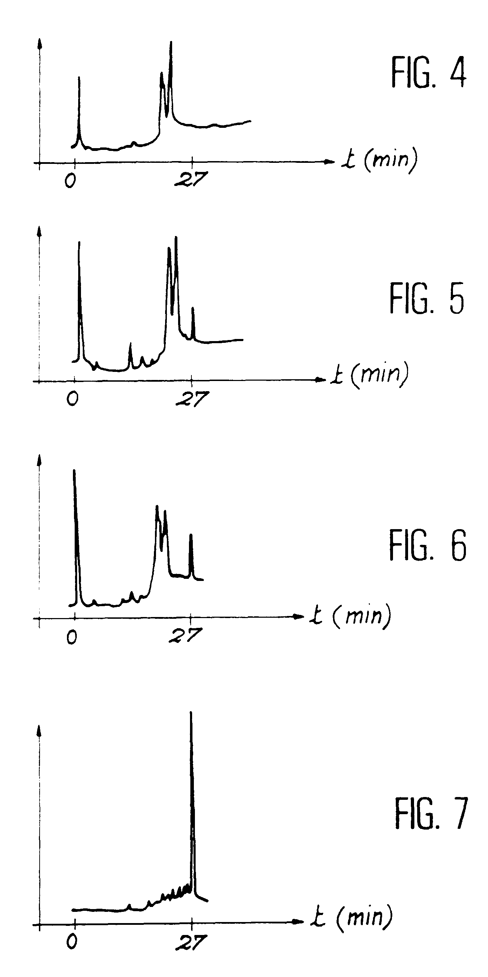

Les résultats obtenus sont donnés sur les figures 4 à 7.

La figure 4 représente le chromatogramme obtenu au temps 0, soit avant réaction avec TEA-3HF.

La figure 5 représente le chromatogramme obtenu après 4h de réaction.

La figure 6 représente le chromatogramme obtenu après 20h de réaction et

La figure 7 représente le chromatogramme obtenu après 20h de réaction lorsqu'on soumet le produit prélevé à un dessalage. Dans tous les cas, on injecte 1µl de produit, sauf dans le cas de la figure 7 où l'on a injecté 2,5µl du produit.

Sur ces chromatogrammes, on remarque que l'oligonucléotide déprotégé apparaît après 4h de réaction (chromatogrammes des figures 5, 6 et 7) pour un temps de rétention t_{R} de 27min. Une déprotection poussée jusqu'à 20h n'amène qu'une faible augmentation de rendement et l'on observe très peu de dégradations supplémentaires (v. fig. 6).

Après dessalage (fig. 7), le produit dessalé montre la pureté de l'oligoribonucléotide brut ; les séquences incomplètes sont bien visibles sur le chromatogramme.

Ainsi, l'oligonucléotide obtenu après déprotection suivie d'un dessalage est très pur et on obtient un rendement important.

### Exemple 5.

Dans cet exemple, on vérifie que le produit TEA-3HF utilisé pour la déprotection, ne dégrade pas les oligoribonucléotides produits en étudiant la stabilité d'un ARN de transfert naturel en présence de TEA-3HF.

A cet effet, on utilise l'ARN de transfert de E. Coli spécifique de la formyl-méthionine (Boehringer) qui contient de nombreuses bases modifiées telles que la 4-thiouridine, la 5-méthyluridine, la dihydrouridine, la pseudouridine, la 7-méthylguanosine et la 2'O-méthylcytidine.

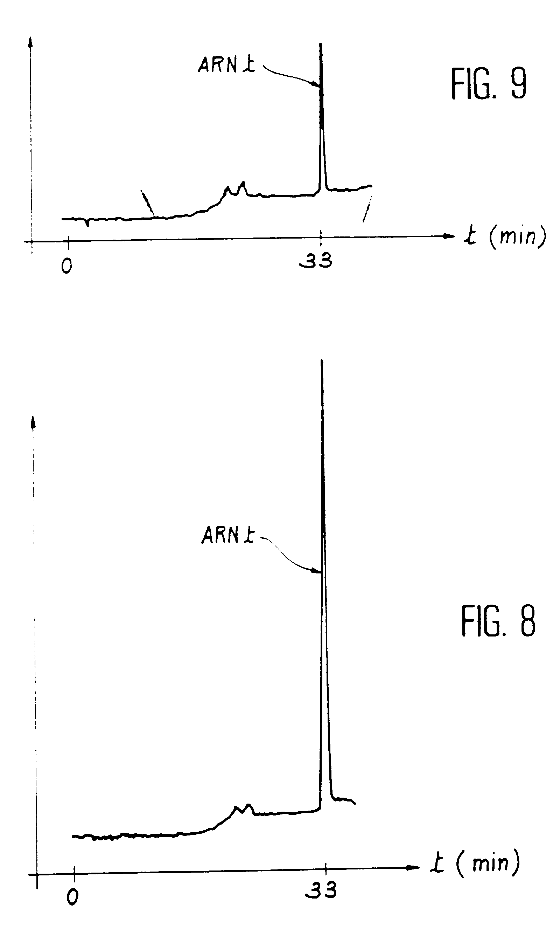

Pour tester la stabilité de cet ARNₜ, on introduit 40µg d'ARNₜ (2 OD) dans 60µl de TEA-3HF et on soumet à une forte agitation au moyen d'un vortex pendant 2h. On laisse ensuite le produit dans ce milieu pendant 20h à la température ambiante, puis on neutralise par 4 volumes de TEAA, 25mM, pH 7,0 et on dessale ensuite le produit sur une colonne NAP-10 (Pharmacia). On récupère 1,8 OD de ce produit (soit 90%) puis on l'analyse par chromatographie liquide à haute performance en échange d'ions comme dans l'exemple 4. La figure 8 représente le chromatogramme obtenu en injectant sur la colonne 0,025 OD.

Sur la figure 9, on a représenté à titre comparatif le chromatogramme obtenu avec le même ARNₜ naturel non traité lorsqu'on injecte sur une colonne identique 0,01 OD.

En comparant ces deux chromatogrammes, on remarque que les temps de rétention sont strictement identiques et qu'aucun produit de séquence incomplète n'est visible sur le chromatogramme de la figure 8.

Par conséquent, l'utilisation du réactif de déprotection TEA-3HF pendant 20h à la température ambiante ne provoque aucune dégradation de l'ARNₜ naturel qui contient des bases modifiées réputées pour leur fragilité.

## Revendications

1. Procédé de synthèse d'acide ribonucléique, caractérisé en ce qu'il consiste
a) à condenser successivement sur un premier nucléoside de formule : 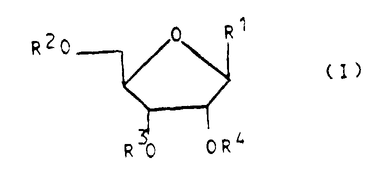 dans laquelle R¹ est un radical dérivé d'une base pyrimidique ou purique choisi parmi les radicaux de formule : 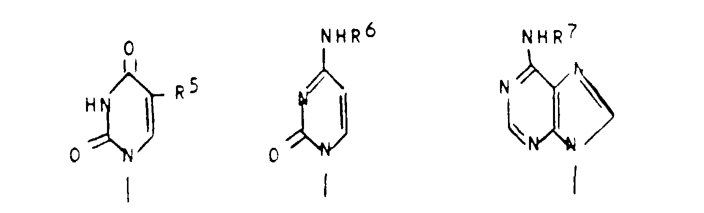 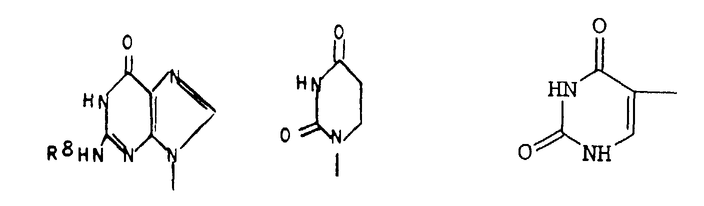 dans lesquelles
R⁵ est H ou CH₃, et R⁶, R⁷ et R⁸ sont des atomes d'hydrogène ou un groupe acyle protecteur d'un groupement NH₂ exocyclique,
R² est un radical labile en milieu acide adapté à la synthèse nucléotidique,
R³ est un support solide, et
R⁴ est un radical trialkylsilyle,
un ou plusieurs nucléosides identiques ou différents répondant à la formule (I) dans laquelle R¹, R² et R⁴ ont la signification donnée ci-dessus et R³ est un radical phosphoré adapté à la synthèse nucléotidique, pour former un acide ribonucléique synthétique dont tous les groupements hydroxyles en 2' sont protégés par R⁴, et
b) à déprotéger tous les hydroxyles en 2' par traitement de l'acide ribonucléique ainsi synthétisé avec un réactif de déprotection répondant à la formule : 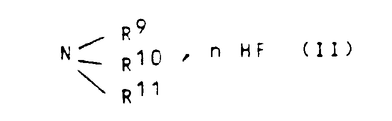 dans laquelle R⁹ représente un radical alkyle linéaire ou ramifié en C₁ à C₁₀, R¹⁰ et R¹¹ qui peuvent être identiques ou différents, représentent H ou un radical alkyle, linéaire ou ramifié, en C₁ à C₁₀ identique ou différent de R⁹, et n est un nombre allant de 1 à 3, entier ou non.

2. Procédé selon la revendication 1, caractérisé en ce que le réactif de déprotection répond à la formule
N(C₂H₅)₃, 3HF

3. Procédé selon l'une quelconque des revendications 1 et 2, caractérisé en ce que l'on réalise le traitement par le réactif de déprotection à la température ambiante pendant 2 à 20h.

4. Procédé selon l'une quelconque des revendications 1 à 3, caractérisé en ce que R⁴ est le radical tertiobutyldiméthylsilyle.

5. Procédé selon l'une quelconque des revendications 1 à 4, caractérisé en ce que R³ répond à la formule 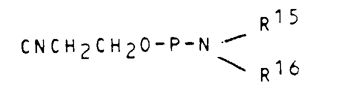 dans laquelle R¹⁵ et R¹⁶ qui sont identiques ou différents sont des radicaux alkyle en C₁ à C₄.

6. Procédé selon la revendication 5, caractérisé en ce que R¹⁵ est le radical méthyle et R¹⁶ est le radical éthyle.

7. Procédé selon la revendication 5, caractérisé en ce que R¹⁵ et R¹⁶ sont des radicaux isopropyle.

8. Procédé selon l'une quelconque des revendications 1 à 7, caractérisé en ce que R² est le radical diméthoxytrityle.

## Patentansprüche

1. Verfahren zur Synthese von Ribonucleinsäure, dadurch gekennzeichnet, daß es darin besteht,
a) nacheinander an ein erstes Nucleosid der Formel: 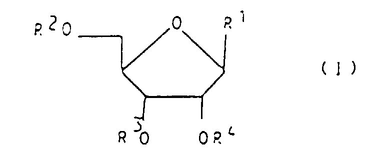 worin R¹ ein von einer Pyrimidin- oder Purinbase abgeleiteter Rest ist, gewählt unter den Resten der Formel: 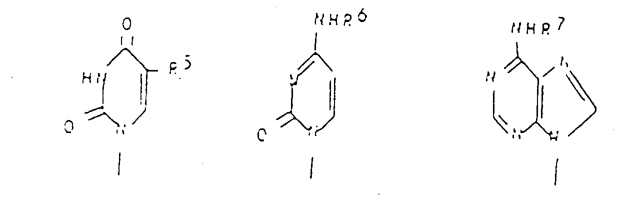 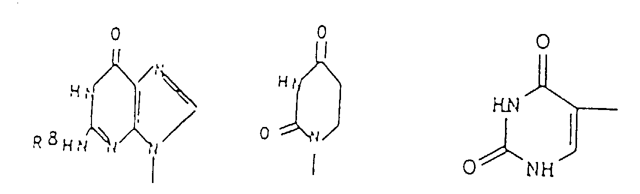 worin
R⁵ H oder CH₃ ist und R⁶, R⁷ und R⁸ Wasserstoffatome oder eine Acyl-Schutzgruppe für eine exocyclische NH₂-Gruppe sind,
R² ein an die Nucleotidsynthese angepaßter, im sauren Medium labiler Rest ist,
R³ ein fester Träger ist und
R⁴ ein Trialkylsilylrest ist,
ein oder mehrere identische oder verschiedene Nucleoside zu kondensieren, die der Formel (I) entsprechen, worin R¹, R² und R⁴ die oben gegebene Bedeutung haben und R³ ein phosphorhaltiger, an die Nucleotidsynthese angepaßter Rest ist, um eine synthetische Ribonucleinsäure zu bilden, bei der alle Hydroxylgruppen in 2'-Stellung durch R⁴ geschützt sind, und
b) an allen Hydroxylgruppen in 2'-Stellung durch Behandlung der so synthetisierten Ribonucleinsäure mit einem Schutzgruppen-Abspaltungsreagens, das der Formel: 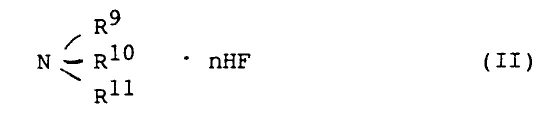 entspricht, worin R⁹ einen linearen oder verzweigten C₁- bis C₁₀-Alkylrest darstellt, R¹⁰ und R¹¹, die identisch oder verschieden sein können, H oder einen linearen oder verzweigten, mit R⁹ identischen oder davon verschiedenen C₁- bis C₁₀-Alkylrest darstellen und n eine ganze oder nichtganze Zahl zwischen 1 und 3 ist, die Schutzgruppe abzuspalten.

2. Verfahren nach Anspruch 1, dadurch gekennzeichnet, daß das Schutzgruppen-Abspaltungsreagens der Formel
N(C₂H₅)₃ · 3HF
entspricht.

3. Verfahren nach einem der Ansprüche 1 und 2, dadurch gekennzeichnet, daß man die Behandlung mit dem Schutzgruppen-Abspaltungsreagens 2 bis 20 Stunden lang bei Umgebungstemperatur ausführt.

4. Verfahren nach einem der Ansprüche 1 bis 3, dadurch gekennzeichnet, daß R⁴ der tert-Butyl-dimethyl-silyl-Rest ist.

5. Verfahren nach einem der Ansprüche 1 bis 4, dadurch gekennzeichnet, daß R³ der Formel 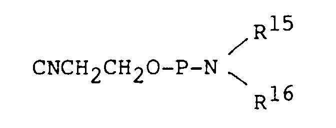 entspricht, worin R¹⁵ und R¹⁶, die identisch oder verschieden sind, C₁- bis C₄-Alkylreste sind.

6. Verfahren nach Anspruch 5, dadurch gekennzeichnet, daß R¹⁵ der Methylrest und R¹⁶ der Ethylrest ist.

7. Verfahren nach Anspruch 5, dadurch gekennzeichnet, daß R¹⁵ und R¹⁶ Isopropylreste sind.

8. Verfahren nach einem der Ansprüche 1 bis 7, dadurch gekennzeichnet, daß R² der Dimethoxytritylrest ist.

## Claims

1. Process for the synthesis of ribonucleic acid, characterized in that it comprises
a) successively condensing on a first nucleoside of formula: 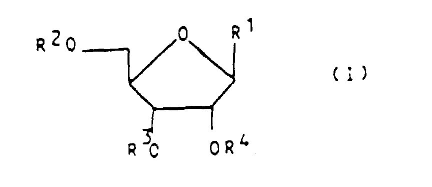 in which R¹ is a radical derived from a pyrimidic or puric base chosen from among the radicals of formula: 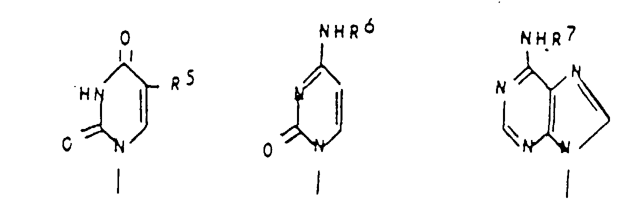 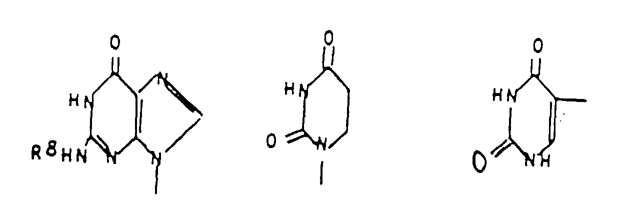 in which
R⁵ is H or CH₃ and R⁶, R⁷ and R⁸ are hydrogen atoms or an acyl group protecting an exocyclic NH₂ group,
R² is an unstable radical in an acid medium appropriate for nucleotide synthesis,
R³ is a solid support and
R⁴ is a trialkyl silyl radical,
one or more identical or different nucleosides complying with the formula (I), in which R¹, R² and R⁴ have the meanings given hereinbefore and R³ is a phosphorus radical appropriate for nucleotide synthesis, in order to form a synthetic ribonucleic acid, whereof all the hydroxyl groups in the 2'-position are protected by R⁴ and
b) deprotecting all the hydroxyls in the 2'-position by treating the thus synthesized ribonucleic acid with a deprotection agent complying with the formula: 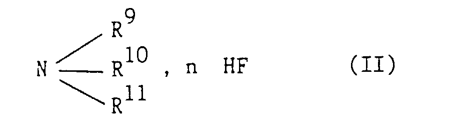 in which R⁹ represents a straight or branched C₁ to C₁₀ alkyl radical, R¹⁰ and R¹¹, which can be the same or different represent H or a straight or branched C₁ to C₁₀ alkyl radical, which can be the same or different to R⁹ and n is a number between 1 and 3, which may or may not be an integer.

2. Process according to claim 1, characterized in that the deprotection reagent complies with the formula
N(C₂H₅)₃, 3HF.

3. Process according to either of the claims 1 and 2, characterized in that the treatment by the deprotection reagent is performed at ambient temperature for 140 min.

4. Process according to any one of the claims 1 to 3, characterized in that R⁴ is the tert. butyl dimethyl silyl radical.

5. Process according to any one of the claims 1 to 4, characterized in that R³ is in accordance with the formula 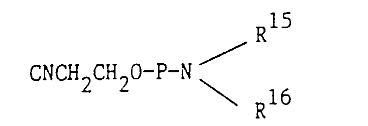 in which R¹⁵ and R¹⁶, which are the same or different, are C₁ to C₄ alkyl radicals.

6. Process according to claim 5, characterized in that R¹⁵ is the methyl radical and R¹⁶ the ethyl radical.

7. Process according to claim 5, characterized in that R¹⁵ and R¹⁶ are isopropyl radicals.

8. Process according to any one of the claims 1 to 7, characterized in that R² is the dimethoxytrityl radical.
